# EUROPEAN PATENT SPECIFICATION

(11) **EP 0 842 290 B1**
(45) Date of publication and mention of the grant of the patent: **28.01.2004**
(21) Application number: 96922367.6
(22) Date of filing: 16.07.1996
(51) Int. Cl.: C12P 7/62, C12P 17/06, C07D 309/30

(54) **PROCESS FOR THE PREPARATION OF LOVASTATIN**
PROZESS FÜR DIE PRÄPARATION VON LOVASTATIN
PROCEDE DE PREPARATION DE LOVASTATINE

(30) Priority: 27.07.1995 WO PCT/SI95/00238
(43) Date of publication of application: 20.05.1998
(73) Proprietor: KRKA, tovarna zdravil, d.d., Novo mesto, 8501 Novo mesto (SI)
(72) Inventor: RADEZ, Ivan, 8000 Novo mesto (SI); BENICKI, Neda, 10000 Zagreb (HR); FILIPOVIC, Branislav, 8000 Novo mesto (SI); ZUPANCIC, Silvia, 11430 Samobor (HR); POKORNY, Miroslav, 8000 Novo mesto (SI); TIHI, Jaroslav, 8000 Novo mesto (SI); KRASOVEC, Dusan, 8232 Sentrupert (SI); ZUPANCIC, Martina, 8270 Krsko (SI)
(74) Representative: UEXKÜLL & STOLBERG
(86) International application number: PCT/SI1996/000016
(87) International publication number: WO 1997/005269

(56) References cited:
- US-A- 4 342 767
- US-A- 5 250 435
- US-A- 5 403 728

## Description

### FIELD OF THE INVENTION

The invention relates to a process for the preparation of lovastatin and derivatives of lovastatin and in particular to a process which involves use of a specific microorganism.

The chemical substance lovastatin exists in two forms, namely the so-called lactone form and the corresponding acid form. The chemical name of the lactone form is 1,2,6,7,8,8a-hexahydro-β,δ-dihydroxy-2,6-dimethyl-8-(2-methyl-1-oxobutoxy)-1-naphthalene heptanoic acid-δ-lactone (Compound I) and the name of the acid form is 1,2,6,7,8,8a-hexahydro-β-δ-dihydroxy-2,6-dimethyl-8-(2-methyl-1-oxobutoxy)-1-naphthalene heptanoic acid (Compound II). The structural formulas are as follows:

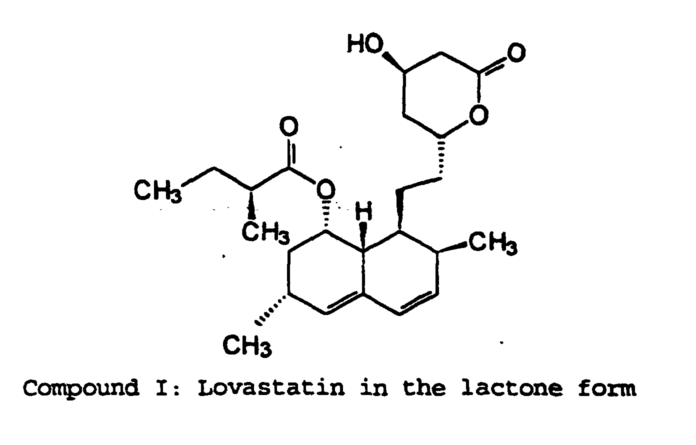

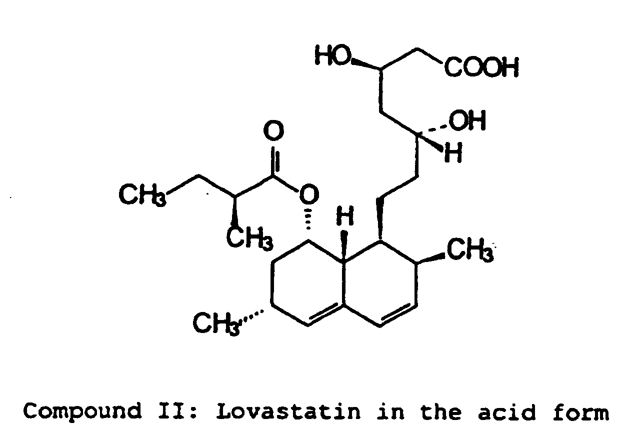

Lovastatin as well as some related substances, e.g. compactin, simvastatin and pravastatin, are known to be effective inhibitors of 3-hydroxy-3-methylglutaryl-coenzyme A reductase (HMG-CoA reductase), which is the key enzyme in the cholesterol biosynthesis (see J.A. Tobert, G. Hitzenberger, W.R. Kuhovatz, I.B. Holmes, K.H. Jones, Atherosclerozis 41, 61-65 (1982)). Lovastatin is in vivo converted from the lactone form (Compound I) into the corresponding acid form (Compound II). The latter is assumed to be the active form of lovastatin. By inhibition of HMG-CoA reductase it is acting as a competitive inhibitor in the biosynthesis of mevalonic acid which is one of the steps in the cholesterol biosynthesis (see G. Zubay, Biochemistry, Addison Wesley Publishing Company, page 584, 1984). Due to this activity, lovastatin is used as a drug to treat hypercholesterolemia and to prevent ischemic cardiac disease. It has been reported in the literature that lovastatin reduces the concentration of the total LDL (Low Density Lipoprotein) and VLDL (Very Low Density Lipoprotein) cholesterol (see S.M. Grundy, G.L. Vega, Journal of Lipid Research 26, 1464-1475 (1985)).

### PRIOR ART

Processes for preparing lovastatin are known in the prior art.

In DE-A-30 06 216 (Sankyo) the preparation of a compound referred to as monacolin K by isolating it as a metabolite of the fungus *Monascus ruber* is disclosed. Later, it was found out that monacolin K and lovastatin are in fact identical substances.

EP-B-22 478 also describes a process for preparing lovastatin. This process makes use of the cultivation of a fungus of the species *Aspergillus terreus*, in particular two deposited strains of this fungus, namely *Aspergillus terreus* ATCC 20541 and *Aspergillus terreus* ATCC 20542.

B.Buckland et al. reported in Novel Microbial Products for Medicine and Agriculture, Elsevier, Amsterdam, pages 161-169, 1989, that by using a reisolate of *Aspergillus terreus* ATCC 20542 an improved process for the production of lovastatin is achieved. It is, however, not disclosed as to how the reisolate can be prepared.

Other fungi which, upon cultivation, may result in lovastatin or related compounds are also disclosed in the art, namely *Penicillium brevicompactum, Pythium ultimum, Hymices chrysospermus, Pacylomyces sp., Eupenicillium sp., Trichoderma* *longibrachiatum, T. pseudokoningii, Phoma sp., Doratomyces nanus, Gymnoascus umbrinus* (see A. Endo, K. Hasumi, A. Yamada, R. Shimoda, H. Takeshima, J. Antibiot. 49, 1609-10 (1986)); *Aspergillus obscurus* (see HU-A-208 997), transformant A.oryzae/A. *terreus* (US-A-5,362,638), as well as *Pleurotus ostreatus, Pleurotus saca* and *Pleurotus sapidus* (see N. Gunde-Cimerman, FEMS Microbiology Letters 11, 203-206 (1993)).

### TECHNICAL PROBLEM

Even though several processes for preparing lovastatin which involve use of different types of microorganisms have been described in the prior art, these known processes suffer from several drawbacks. Firstly, the lovastatin producing microorganisms which are employed in these processes do not give satisfactory yields of lovastatin. Moreover, during their cultivation it is often necessary to feed up the fermentation mixture during the process, in particular with the selected carbon source, since a high initial concentration of the carbon source tends to have a negative effect on the production rate of lovastatin. Finally, the necessity in known processes to use a feeding up during the fermentation process involves the risk that the culture broth may be contaminated.

### DESCRIPTION OF THE INVENTION

The aforementioned disadvantages are overcome by the process according to the present invention. This process for the preparation of lovastatin in the lactone form (Compound I) and in the acid form (Compound II) as defined by the following formulas 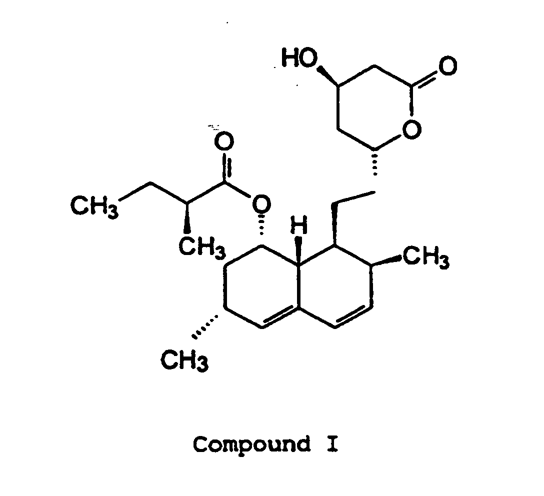 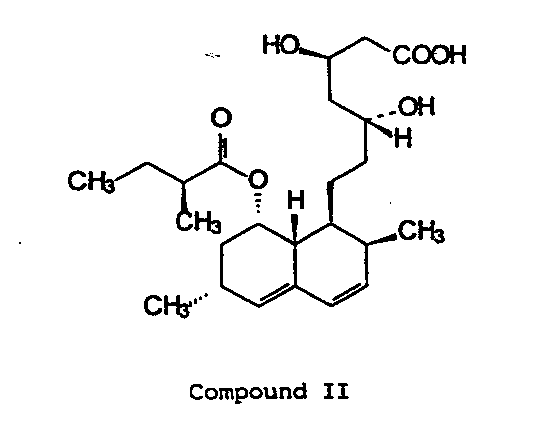 or of pharmaceutically acceptable salts or alkyl esters thereof comprises fermenting a nutrient medium with a microorganism of the species *Aspergillus terreus* var. *aureus* and isolating the desired product.

It was unexpectedly found that by using a microorganism of the species *Aspergillus terreus* var. aureus excellent yields of lovastatin can even be achieved if a high concentration of carbon source is present in the medium at the beginning of the fermentation process. Therefore, the undesirable feeding up of the fermentation broth is not necessary.

The genus *Aspergillus*, which includes also the *Aspergillus terreus* microorganisms used in the process according to the invention, is a very big and heterogeneous taxonomic category. It is split up in subgenuses and sections or groups and even specific varieties. In the literature several varieties of the fungus *Aspergillus terreus*, such as *Aspergillus terreus* var. *globosus, Aspergillus terreus* var. *terreus, Aspergillus terreus* var. au*reus, Aspergillus terreus var. africanus*, are described (see K.B. Raper and D. Fenell: The genus *Aspergillus,* Williams and Wilkins, Baltimore, 1965). Further, in the International microbiological collections so-called "type strains" are available for the mentioned varieties.

It was now found that a specific fungus, which was determined as *Aspergillus terreus* var. *aureus*, is a very advantageous lovastatin-producing microorganism. A culture of a particularly preferred *Aspergillus terreus* var. *aureus* microorganism is deposited with the Belgian Coordinated Collections of Microorganisms - Mycothèque de l'Universite Catholique de Louvain under accession number MUCL 38997.

For the taxonomic identification of this microorganism there were used type strains deposited in the Culture Collections according to the Budapest Treaty as well as standard reference books, e.g. K.B. Raper and D. Fenell: The genus *Aspergillus*, Williams and Wilkins, Baltimore, 1965, R.R.M. Paterson and P.D. Bridge: Biochemical Technique for Filamentous Fungi, IMI Technical Handbooks, No. 1, Cab International 1994 and M.J. Carlile and S.C. Watkinson: The Fungi, Academic Press 1994.

The cultivation of the microorganisms used in the process according to the invention on a Czapek agar (CZA) was carried out at 28°C for 14 days. The colonies obtained were 5 to 7 cm in diameter. The mycelium was pale yellow with intensive or poor sporulation. The conidial heads were long, globose, compact, 110 to 250 µm in length and 35 to 60 µm in diameter. The conidiophores were more or less curved, smooth, colorless up to pale yellow, 300 to 400 µm in length, and 4 to 7 µm in diameter. The vesicles were hemispherical. Primary sterigmata were 5.5 to 7 µm in length and 1 to 2 µm in diameter, and secondary sterigmata were 5 to 7 µm in length and 1.5 to 2 µm in diameter. The conidia were globose to slightly elliptical, smooth, and 1.9 to 2.4 µm in diameter. In growing on various substrates, pigment having a yellow to green-brown color was released into the culture medium.

The taxonomic identification revealed that the microorganisms used in the present invention belonged to the class of Ascomycetes - higher fungi, family Eurotiaceae, genus Eurotium, and conidial form *Aspergillus*, species *Aspergillus terreus* var. aureus, respectively. Since *Aspergillus* terreus ATCC 20542 is the strain which has most often been mentioned in the prior art as lovastatin producer, the microorganism preferably used in the process according to the invention was also compared with this prior art microorganism. The result of an extensive comparative analysis based on a morphological analysis, the physiology of growth in various nutrient media and, the pigment analysis confirmed that the lovastatin producer *Aspergillus terreus var aureus* MUCL 38997 according to the invention is substantially different from the strain *Aspergillus terreus* ATCC 20542. For that purpose several cultivation experiments of both fungal strains were conducted at a temperature of 30°C, and for 10 to 12 days in the dark.

The results of these experiments are given in Table 1 and Table 2.

**TABLE 1**

| Macromorphological comparison of the fungal strains *Aspergillus terreus* var*, aureus* MUCL 38997 and *Aspergillus terreus* ATCC 20542, producers of lovastatin, grown on synthetic. solid culture media containing different carbon sources. | | |
|---|---|---|
| **Carbon source in medium** | *Aspergillus terreus* var. *aureus* MUCL 38997 (Invention) | *Aspergillus terreus* ATCC 20542 (Prior Art) |
| **Xylose** | Regular shape colony, diameter 9.5 cm, with yellow patches. Aerial mycelia, cotton-like, white to light yellow. Substrate mycelia mature. Soluble pigment: none. | Regular shape colony, diameter 8 cm, light rust brown with white margin. Aerial mycelia, powder-like. Soluble pigment: yellow. |
| **Saccharose** | More or less regular shape colony, diameter 8 cm, pale ocher. Aerial mycelia | Irregular colony, lobe shape, diameter 6 cm, with 4 zones: 1) central zone white-yellow;tubular |
| | cotton-like, pale yellow. Substrate mycelia median maturity. Soluble pigment: none. | 2) white zone tufted. 3) light rust brown with radial furrows, velvety. 4) margin white ocher. Soluble pigment: yellow-brownish. |
| **Rhamnose** | Regular shape colony, diameter 9 cm, rich ocher-yellow. Soluble pigment: yellow. | Slitted lobe colony, diameter 5 cm, with 2 zones: 1) central zone: diameter 3 cm, rust brown, 2) external zone: ocher. Soluble pigment: none. |

| **Carbon source in medium** | *Aspergillus terreus* var. *aureus* MUCL 38997 | *Aspergillus terreus* ATCC 20542 |
|---|---|---|
| **Ribose** | Regular shape colony, whity with intermediate zone pure yellow. Soluble pigment: none. | Regular shape colony, diameter 8 cm, with 2 zones: 1) central zone: ocher, diameter 3 cm, with furrows, 2) external zone: pale ocher-yellow, margin whity Soluble pigment: none. |
| **Maltose** | Regular-shape colony, diameter 9 cm, with 2 zones: 1) central zone: flatted diameter 7 cm, whitish-ocher with yellow centre, 2) external zone: tufted white-ocher. | Irregular shape colony, diameter 7-8 cm, ocher, with 4 zones: 1) central zone: diameter 3 cm, whity 2) diameter zone: 0.5 cm, tufted, pale ocher. 3) diameter zone: 1.5 cm, rust brown, velvety, |
| | Soluble pigment: none. | 4) margin pale ocher. Soluble pigment: none. |
| **Fructose** | Regular shape colony, diameter 9 cm, yellowish-white. Soluble pigment: none. | More or less regular shape colony; diameter 8 cm, with 3 zones: 1) central zone: diameter 5 cm, white-ocher, tufted and furrowed, 2) 1 cm diameter zone, white rust brown, velvety, 3) margin pale ocher. Soluble pigment: yellow. |
| **Arabinose** | Regular shape colony, whitish-ocher, diameter 10 cm, Soluble pigment: orange-yellow. | Regular shape colony, diameter 10 cm, ocher, Soluble pigment: brown. |

**TABLE 2**

| Macromorphological comparison of the fungal strains *Aspergillus terreus* var. *aureus* MUCL 38997 and *Aspergillus terreus* ATCC 20542, producers of lovastatin, grown on synthetic, solid culture media containing different nitrogen sources. | | |
|---|---|---|
| **Nitrogen source in medium** | *Aspergillus terreus* var. *aureus* MUCL 38997 (Invention) | *Aspergillus terreus* ATCC 20542 (Prior Art) |
| **Glycerol and alanine** | Regular shape colony, diameter 9 cm, pale ocher. External margin 1 cm, ocher, Soluble pigment: yellow. | Irregular shape colony, diameter 8 cm, Central zone diameter 3 cm, pale ocher, rest of colony dark ocher. Soluble pigment: brown. |
| **Glycerol and L-isoleucine** | Regular shape colony, diameter 9 cm, white. Soluble pigment: none. | Irregular shape colony, diameter 8 cm, with 3 zones: 1) central zone: diameter 4 cm, pale beige white, tufted 2) diameter 1 cm, |

| **Nitrogen source in medium** | *Aspergillus terreus* var. *aureus* MUCL 38997 | *Aspergillus terreus* ATCC 20542 |
|---|---|---|
| | | ocher. 3) external zone: diameter 1 cm. pale ocher. Soluble pigment: brown. |

As evident from the results given in the above Tables 1 and 2, the strains differ markedly in coloration of colonies and penetration of pigment into the culture medium.

In most substrates, the aerial mycelium of the fungus *Aspergillus terreus* var. *aureus* MUCL 38997 is white to pale yellow, and the substrate mycelium is pale brown. Intensively yellow pigment was observed when arabinose or rhamnose were used as carbon source. Under the same conditions, the color of the microorganism *Aspergillus terreus* ATCC 20542 is rust brown. With respect to colonies sizes it was found that in most carbon sources tested, namely xylose, saccharose, maltose, rhamnose, ribose, fructose and arabinose, the colonies of *Aspergillus terreus* var. aureus MUCL 38997 were 9 cm in diameter. Upon cultivation of the fungal strain *Aspergillus terreus* ATCC 20542 under the same growing conditions, the colonies were in most cases only 5 to 8 cm in diameter.

Utilisation of sodium citrate as the carbon source hindered the growth of *Aspergillus terreus* var. *aureus* MUCL 38997. The opposite effect was observed in case of *Aspergillus terreus* ATCC 20542 where, after 12 days of cultivation, the colonies of this strain grew in more or less irregular shape and brown color and velvety structure.

By use of various nitrogen sources, such as asparagine, glutamic acid, valine, leucine, L-isoleucine, arginine, alanine, tyrosine, NH₄Cl and calcium nitrate, a stimulating effect on colonies growth of the *Aspergillus terreus* var. *aureus* MUCL 38997 strain was observed. There were obtained right-shape colonies with well developed aerial and substrate mycelium. In the case of *Aspergillus terreus* ATCC 20542 the opposite effect was observed when asparagine and tyrosine were used as nitrogen sources, namely that the growth was inhibited.

Further, by means of microscopy the so-called "hülla cells" were not observed in the case of *Aspergillus terreus* var. *aureus* MUCL 38997, which is typical for var. *aureus*. In contrast to this, "hülla cells" were present in the case of *Aspergillus terreus* ATCC 20542.

Thus, the above results show that MUCL 38997 as used in the present process and ATCC 20542 are completely different microorganisms.

The cultivation of the microorganisms used in the process according to the invention is performed in aqueous media for example those which are used for the preparation of other fermentation products. The fermentation is usually carried out in media containing assimilable carbon and nitrogen sources as well as mineral salts and is preferably carried out in the submerged state and under aerobic conditions as the microorganisms show a very fast growth under such conditions. It is particularly preferred that high initial concentrations of the selected carbon source are present in the fermentation medium, whereby the need for further feeding up with carbon source during the fermentation process does not arise. Initial concentrations of carbon source are therefore usually ≥ 6 wt.%, preferably ≥ 9 wt.%, more preferably ≥ 11 wt.% and most preferably ≥ 15 wt.% of the medium.

Examples of suitable components of the nutrient media are described in e.g. EP-B-22 478. Especially preferred carbon sources are lactose, glucose, saccharose, malt flour and particularly preferred nitrogen sources are casein-peptone, corn steep liquor (CSL), soya-protein, yeast extract and brewer's yeast. Particularly preferred salts included in the nutrient medium are MgCl₂, K₂HPO₄, NaH₂PO₄, MnSO₄, (NH₄)₂HPO₄. The nutrient medium may also contain other desirable components, such a defoaming agents.

The fermentation process is preferably carried out at temperatures in the range from 20 to 33°C, in particular at about 28°C. The pH-value of the nutrient medium will normally be in the range of 6.0 to 8.0, and in particular 5.8 to 6.8. The completion of the fermentation usually takes about 6 days. It was found out that lovastatin was produced mainly in the acid form while the concentration of the lactone form in the fermentation broth remained fairly low during the whole process. Lovastatin was isolated by conventional processes. Typically isolation is carried out by extraction with organic solvents, lactonization and crystallization. The isolated lovastatin was characterized by several analytical techniques, such as IR-spectrometry and mass spectrometry, NMR and UV spectrometry.

The acid form of lovastatin (Compound II) may be lactonized to the corresponding lactone form (Compound I) by conventional processes as are described in e.g. EP-A-351 918, EP-B-22 478 or WO 94/29292. The obtained lovastatin can also be converted into the corresponding pharmaceutically acceptable salts or alkyl esters by means of conventional methods such as those described in EP-B-22 478.

It was further discovered that the process according to the invention resulted in higher yields of lovastatin compared with prior art processes. Further, even at high initial concentrations of the carbon sources used, the microorganism used in the present process showed a high productivity and accordingly it was not necessary to add further carbon source during the process thereby avoiding the risk of contamination of the fermentation broth.

The superiority of the present process is also shown by the following examples which illustrate the invention without limiting the scope of the invention.

### EXAMPLES

### Example 1 - General procedure

For this example a sample of the fungus *Aspergillus terreus* var. *aureus*, which is deposited under Accession number MUCL 38997 is used. A well sporulated culture of this fungus obtained by cultivation on potato-dextrose agar (PDA) was used to prepare a spore suspension and this spore suspension was employed to inoculate the medium used for the growth of the vegetative phase. This medium had a pH of 6.4 and contained corn steep liquor (CSL), malt flour, casein peptone, brewer's yeast, (NH₄)₂HPO₄, a defoaming agent and drinking water. Prior to the inoculation with the spore suspension the medium was sterilized at 121°C for 20 minutes and subsequently cooled to 25-35°C, preferably 28°C.

The inoculated prefermentor culture was allowed to grow at 28°C for 20 to 35 hours with aeration and agitation being applied.

Well developed inoculum (1 to 2% by volume) was then transferred under sterile conditions into the fermentation medium. The fermentation medium contained carbon sources and nitrogen sources as well as mineral salts, namely MgCl₂, K₂HPO₄, NaH₂PO₄, MnSO₄, and water. The fermentation process was run at 28°C with aeration and mixing being applied depending on the concentration of dissolved oxygen. During the fermentation the pH was maintained between 5.8 to 6.8 by using 30 % H₂SO₄ or 10 % NaOH solution.

During the fermentation, the lovastatin concentration in the culture broth was measured by HPLC. For that purpose a sample of the broth was acidified to a pH value of 4 with 10 % aqueous HCl, extracted with methanol, homogenized with the Ultra Turax T25 homogenizator (Janke und Kunkel, IKA Labortechnik, Germany) for 5 minutes, and filtered. The filtrate was used for the analysis by means of HPLC (Apparatus: Pharmacia LKB system; Column: Chrompack Chromspher C18, 5 µm; temperature + 45°C: Eluent: acetonitrile and 0.1 % aqueous phosphoric acid (volume ratio of 48:52); UV detector.

It was found that mainly lovastatin in the acid form was produced, while the lactone concentration remained considerably low during the whole fermentation process.

The fermentation was completed after about 6 days and lovastatin was isolated according to the method disclosed in WO 94/29292. IR-spectroscopy, mass spectrometry as well as NMR and UV spectroscopy confirmed that lovastatin was formed.

### Example 2

Mycelium of the fungus *Aspergillus terreus* var. *aureus* MUCL 38997 was grown on potato-dextrose agar (PDA) for 10 to 14 days. The mycelium was then scraped off and used to prepare a spore suspension in an aqueous 0.1 % by volume Tween-80 solution. 200 ml of the filtered spore suspension were subsequently employed to inoculate 30 1 of the medium for the vegetative growth (vegetative medium) which had the following composition.

| Composition of the vegetative medium: | |
|---|---|
| CSL | 5.0 g |
| malt flour | 15.0 g |
| casein peptone | 5.0 g |
| brewer's yeast | 1.2 g |
| (NH₄)₂HPO₄ | 0.015 g |
| drinking water | up to 1000 ml |

The pH of the medium was adjusted to 6.4 before sterilization. The sterilization was carried out at 121°C for 20 minutes. The vegetative broth was allowed to grow with aeration and mixing at 28°C for 20 to 35 hours. 1 % by volume of the broth obtained was transferred aseptically into the fermentation medium (100 1), sometimes also referred to as production medium, of the following composition:

| Composition of the fermentation medium: | |
|---|---|
| lactose | 60 g |
| organic nitrogen source | 13.8 g |
| CSL | 1.95 g |
| MgCl₂.6 H₂O | 1.0 g |
| NaH₂PO₄ | 1.2 g |
| K₂HPO₄ | 0.5 g |
| MnSO₄.H₂O | 0.02 g |
| defoaming agent | 0.5 g |
| drinking water | up to 1000 ml |

Before inoculation, the pH value of the production medium was adjusted to 6.4 and it was sterilized. The sterilization was conducted at 121°C for 20 minutes.

The culture was allowed to grow at 28°C with aeration and mixing for 6 days. The production of lovastatin was monitored by HPLC and after completion of the fermentation the lovastatin produced was isolated as described in example 1. A yield of more than 500 µg lovastatin in the acid form per ml of culture broth was obtained.

### Example 3

The process according to example 2 was repeated up to the stage where the growth of the vegetative phase was completed.

2 % by volume of the so-obtained culture broth were then used to inoculate 100 1 of a fermentation medium having the following composition:

| Composition of the fermentation medium: | |
|---|---|
| lactose | 90 g |
| organic nitrogen source | 22.15 g |
| CSL | 2.93 g |
| MgCl₂.6 H₂O | 1.0 g |
| NaH₂PO₄ | 1.2 g |
| K₂HPO₄ | 0.5 g |
| MnSO₄.H₂O | 0 . 02 g |
| defoaming agent | 0.5 g |
| drinking water | up to 1000 ml |

Before inoculation, the pH value of the fermentation medium was adjusted to 6.4 and it was sterilized. The sterilization was conducted at 121°C for 20 minutes.

The culture was allowed to grow, with aeration and mixing at a temperature of 28°C for 6 days. The lovastatin produced was then recovered as described in example 1. The yield was more than 700 µg/ml lovastatin in the acid form.

### Example 4

The process according to example 2 was repeated up to the stage where the growth of the vegetative phase was completed.

2 % by volume of the so-obtained culture broth were then used to inoculate 100 1 of a fermentation medium having the following composition:

| Composition of the fermentation medium: | |
|---|---|
| lactose | 117 g |
| organic nitrogen source | 28.8 g |
| CSL | 3.81 g |
| MgCl₂.6 H₂O | 1.0 g |
| NaH₂PO₄ | 1.2 g |
| K₂HPO₄ | 0.5 g |
| MnSO₄.H₂O | 0.02 g |
| defoaming agent | 0.5 g |
| drinking water | up to 1000 ml |

Before inoculation, the pH value of the fermentation medium was adjusted to 6.4 and it was sterilized. The sterilization was conducted at 121°C for 20 minutes.

The culture was allowed to grow, with aeration and mixing at a temperature of 28°C for 6 days. The lovastatin produced was then recovered as described in example 1. The yield was more than 1000 µg/ml lovastatin in the acid form.

### Example 5

The process according to example 2 was repeated up to the stage where the growth of the vegetative phase was completed.

2 % by volume of the so-obtained culture broth were then used to inoculate 100 1 of a production medium having the following composition:

| Composition of the production medium: | |
|---|---|
| lactose | 150 g |
| organic nitrogen source | 35.14 g |
| CSL | 4.65 g |
| MgCl₂.6 H₂O | 1.0 g |
| NaH₂PO₄ | 1.2 g |
| K₂HPO₄ | 0.5 g |
| MnSO₄.H₂O | 0.02 g |
| defoaming agent | 0.5 g |
| drinking water | up to 1000 ml |

Before inoculation, the pH value of the production medium was adjusted to 6.4 and it was sterilized. The sterilization was conducted at 121°C for 20 minutes.

The culture was allowed to grow, with aeration and mixing at a temperature of 28°C for 6 days. The lovastatin produced was then recovered as described in example 1. The yield was more than 1200 µg/ml lovastatin in the acid form.

### Example 6 (comparative)

### Comparison of productivity of strains A. terreus ATCC 20542 and A. terreus var. aureus MUCL 38997

To compare the productivity of the microorganism preferably used in the process according to the invention with the prior art microorganism *A. terreus* ATCC 20542, the following experiments were performed on a laboratory scale.

In all experiments the composition of the seed media used for the growth of the vegetative phase was the same as described in example 2. To determine the influence of different concentrations of carbon source, the production media according to examples 2, 3 and 4, respectively, were used. These media contain 6 % (example 2), 9 % (example 3) and 11.7 % (example 4) of lactose. Sterilization of the media was in all experiments carried out at 121°C for 20 minutes.

The inoculation of the medium for the growth of the vegetative phase was conducted as described in example 2. The seed flasks were incubated at 28°C on a 220 rpm shaker (5 cm throw) . Subsequently 2 % by volume of the culture broth obtained were aseptically transferred into 500 ml Erlenmayer flasks containing 60 ml of the respective production medium. Inoculated flasks were then incubated 28°C (rpm = 220, 5 cm throw) for 140 hours. The production of lovastatin was controlled by HPLC. The respective yields of lovastatin (µg/ml) are given in the table below.

| hours | 6 % lactose | | 9 % lactose | | 11.7 % lactose | |
|---|---|---|---|---|---|---|
| | 20542 | 38997 | 20542 | 38997 | 20542 | 38997 |
| 96 | 298 | 1228 | 176 | 1242 | 94 | 1156 |
| 140 | 318 | 1320 | 200 | 1804 | 126 | 1378 |

(20542 refers to ATCC 20542
38997 refers to MUCL 38997).

From the results presented in the above table it becomes clear that the strain used in the process according to the invention shows a much higher productivity than the prior art strain tested. Further, the strain employed in the present process is capable of producing high yields of lovastatin even under very high initial concentrations of carbon source, whereas the productivity of the prior art strain is inhibited by such concentrations.

Therefore, these test results show the surprising superiority of the microorganism employed in process according to the invention.

### Example 7 (comparative)

### Comparison of productivity of strains A. terreus ATCC 20542 and A. terreus var. aureus MUCL 38997

Experiments were performed on a laboratory scale in accordance with the procedures described in EP-B-22478, example 5.

The described procedures were repeated with the exception that the strains *A. terreus* ATCC 20542 and *A. terreus* var. *aureus* MUCL 38997 were used as strains. The lovastatin yields (µg/ml) for these two strains are given in the table below.

| | Medium for growth of vegetative phase and production medium as in example 5 of EP-B-22 478 | |
|---|---|---|
| hours | 20542 | 38997 |
| 120 | 114 | 184 |
| 144 | 172 | 222 |
| 168 | 150 | 214 |

(20542 refers to ATCC 20542 and
38997 refers to MUCL 38997)

The above data also show that also under these conditions the strain used in the process according to the invention proves to have a much higher productivity than the prior art strain.

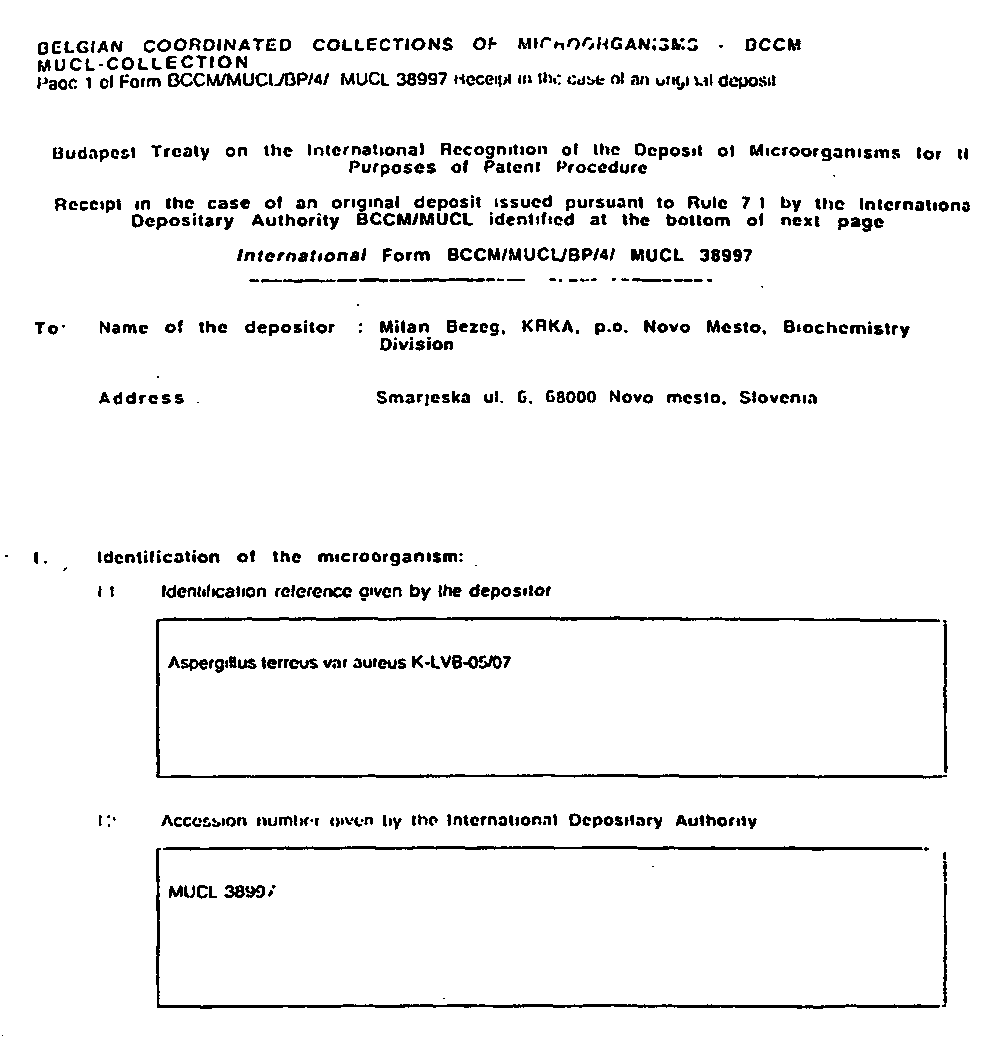

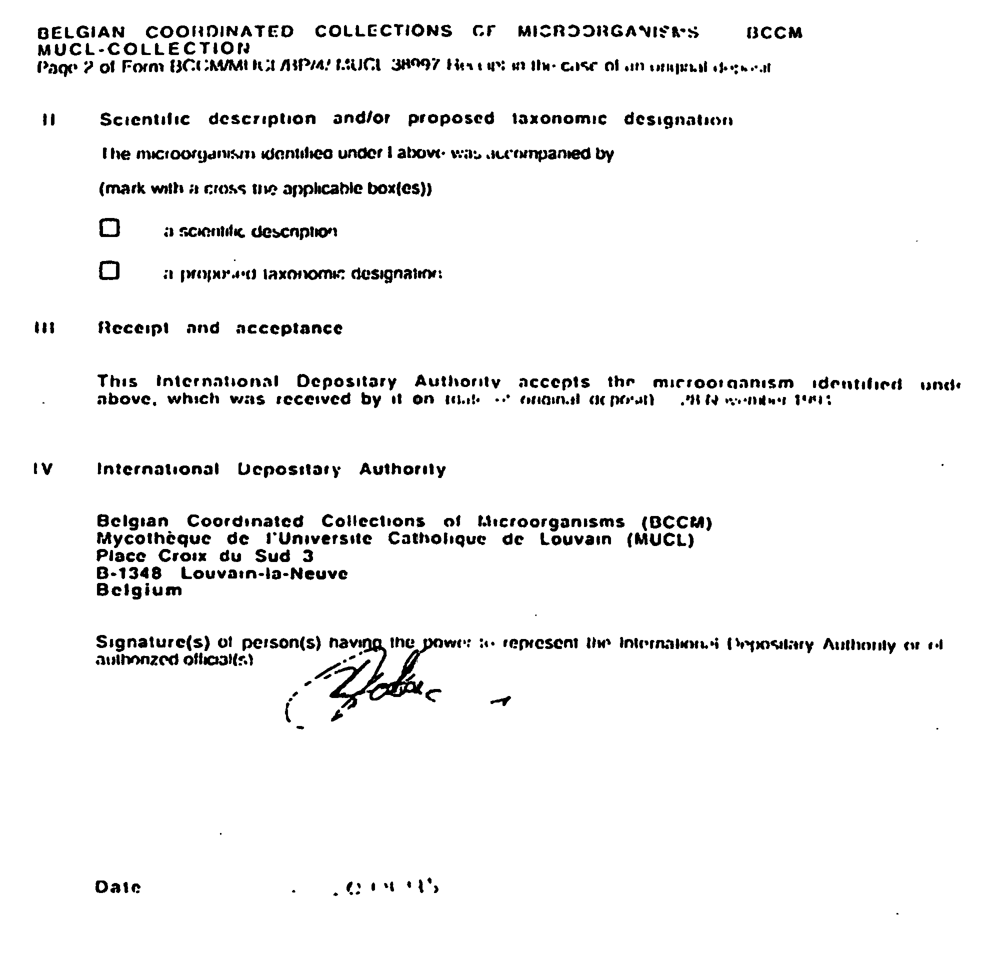

## Claims

1. A process for the preparation of compound I and compound II as defined by the following formulas 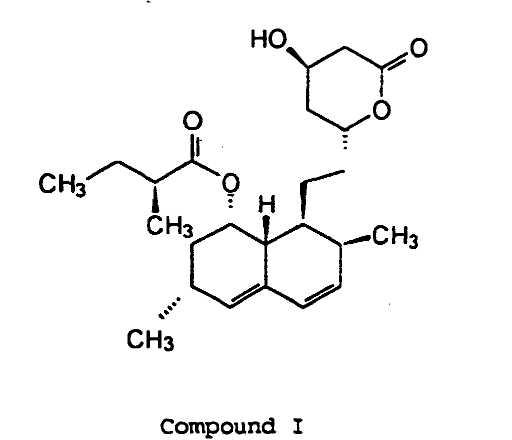 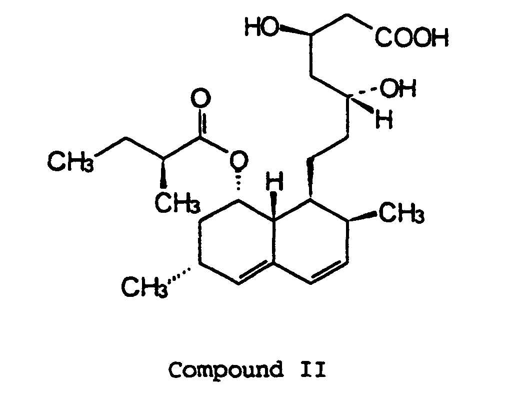 or pharmaceutically acceptable salts or alkyl esters thereof, which comprises fermenting a nutrient medium with a microorganism of the species *Aspergillus terreus* var. aureus and recovering the desired product.

2. Process according to claim 1, wherein the microorganism deposited with the Belgian Coordinated Collections of Microorganisms - Mycothèque de l'Université Catholique de Louvain under Accession Number MUCL 38997 is used.

3. Process according to claim 1 or 2, wherein a nutrient medium is used which contains ≥ 6 wt.% and in particular ≥ 9 wt.% of carbon source.

4. Process according to claim 3, wherein a nutrient medium is used which contains ≥ 11 wt.% and in particular ≥ 15 wt.% of carbon source.

## Patentansprüche

1. Verfahren zur Herstellung von Verbindung I und Verbindung II, definiert durch die folgenden Formeln 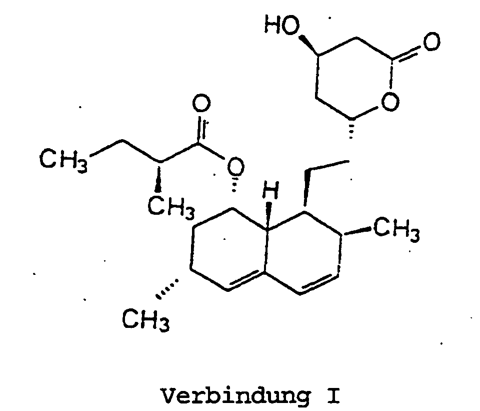 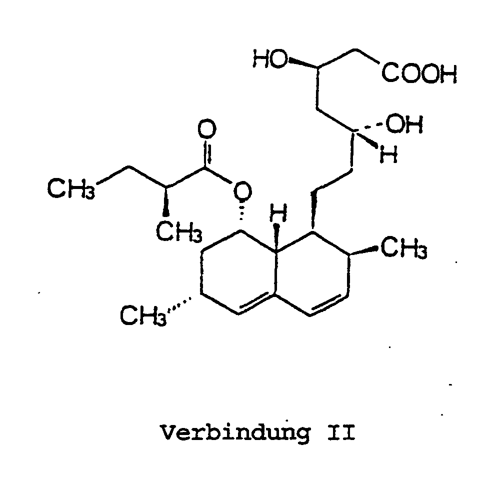 oder pharmazeutisch akzeptabler Salze oder Alkylester davon,
das die Fermentation einer Nährlösung mit einem Mikroorganismus der Art *Aspergillus terreus* var. *aureus* und die Gewinnung des gewünschten Produktes umfasst.

2. Verfahren nach Anspruch 1, bei dem man den Mikroorganismus verwendet, der bei der Belgischen Koordinierten Sammlung von Mikroorganismen - Mycothèque de l'Université Catholique de Louvain unter der Zugangsnummer MUCL 38997 - hinterlegt ist.

3. Verfahren nach Anspruch 1 oder 2, bei dem man eine Nährlösung verwendet, die ≥ 6 Gew.%, insbesondere ≥ 9 Gew.% einer Kohlenstoffquelle enthält.

4. Verfahren nach Anspruch 3, bei dem man eine Nährlösung verwendet, die ≥ 11 Gew.%, insbesondere ≥ 15 Gew.% einer Kohlenstoffquelle enthält.

## Revendications

1. Procédé pour la préparation d'un composé I et d'un composé II, comme définis par les formules suivantes, 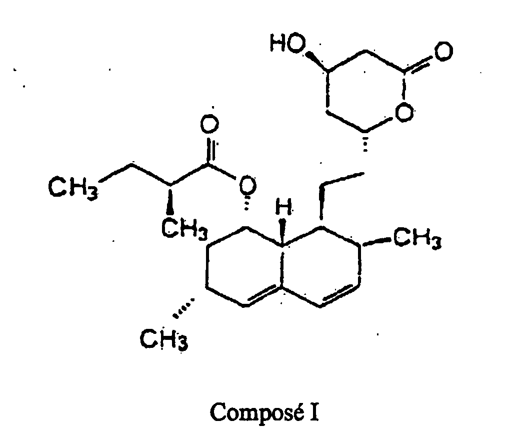 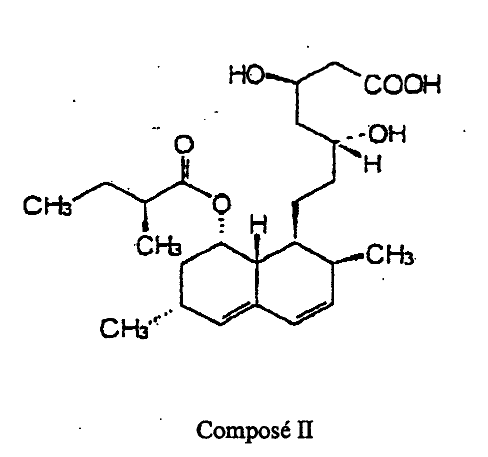 ou de sels ou esters alkyliques pharmaceutiquement acceptables de ceux-ci comprenant la fermentation d'un milieu nutritif avec un microorganisme de l'espèce *Aspergillus terreus* var. *aureus* et la récupération du produit désiré.

2. Procédé selon la revendication 1, dans lequel on utilise le microorganisme déposé auprès des Collections coordonnées belges de microorganismes - Mycothèque de l'Université Catholique de Louvain, sous le numéro de dépôt MUCL 38997.

3. Procédé selon la revendication 1 ou 2, dans lequel est utilisé un milieu nutritif contenant ≥ 6% en poids et, en particulier ≥ 9% en poids, d'une source de carbone.

4. Procédé selon la revendication 3, dans lequel est utilisé un milieu nutritif contenant ≥11% en poids et, en particulier ≥15% en poids, d'une source de carbone.
